# EUROPEAN PATENT SPECIFICATION

(11) **EP 1 774 340 B1**
(45) Date of publication and mention of the grant of the patent: **29.10.2008**
(21) Application number: 05776018.3
(22) Date of filing: 19.07.2005
(51) Int. Cl.: G01N 33/68, G01N 33/50

(54) **INSULIN PROMOTER FACTOR 1 AS TARGET/MARKER OF BETA CELL FAILURE**
INSULINPROMOTOR-FAKTOR 1 ALS TARGET/MARKER VON BETA-ZELLEN-STÖRUNG
FACTEUR 1 DU PROMOTEUR D'INSULINE (IPF-1) UTILISE EN TANT QUE CIBLE/MARQUEUR DE LA DEFAILLANCE DES CELLULES BETA

(30) Priority: 28.07.2004 EP 04103611
(43) Date of publication of application: 18.04.2007
(73) Proprietor: F. Hoffmann-La Roche AG, 4070 Basel (CH)
(72) Inventor: CHRIST, Andreas, CH-4144 Arlesheim (CH); EVERS, Stefan, 79379 Muellheim (DE); KRAPFENBAUER, Kurt, A-1200 Wien (AT); SEBOKOVA, Elena, CH-4103 Bottmingen (CH)
(74) Representative: Küng, Peter
(86) International application number: PCT/EP2005/007854
(87) International publication number: WO 2006/010530

(56) References cited:
- WO-A-20/04022772
- WO-A1-20/04027430
- WO-A2-03/061583
- US-A1- 2004 005 299
- ZANGEN D H ET AL: "Reduced insulin, GLUT2, and IDX-1 in beta-cells after partial pancreatectomy." DIABETES. FEB 1997, vol. 46, no. 2, February 1997 (1997-02), pages 258-264, XP008055761 ISSN: 0012-1797
- ROBERTSON R PAUL ET AL: "Glucose toxicity in beta-cells: type 2 diabetes, good radicals gone bad, and the glutathione connection." DIABETES. MAR 2003, vol. 52, no. 3, March 2003 (2003-03), pages 581-587, XP002354480 ISSN: 0012-1797
- PROCHIANTZ A: "Messenger proteins: homeoproteins, TAT and others." CURRENT OPINION IN CELL BIOLOGY. AUG 2000, vol. 12, no. 4, August 2000 (2000-08), pages 400-406, XP002956397 ISSN: 0955-0674
- NOGUCHI HIROFUMI ET AL: "PDX-1 protein containing its own antennapedia-like protein transduction domain can transduce pancreatic duct and islet cells." DIABETES. JUL 2003, vol. 52, no. 7, July 2003 (2003-07), pages 1732-1737, XP002354481 ISSN: 0012-1797
- REIMER MARTINA KVIST ET AL: "Altered beta-cell distribution of pdx-1 and GLUT-2 after a short-term challenge with a high-fat diet in C57BL/6J mice." DIABETES. FEB 2002, vol. 51 Suppl 1, February 2002 (2002-02), pages S138-S143, XP002354482 ISSN: 0012-1797
- BERGER B.; STENSTROM G.; SUNDKVIST G.: 'Random C-peptide in the classification of diabetes' SCANDINAVIAN JOURNAL OF CLINICAL AND LABORATORY INVESTIGATION vol. 60, no. 8, December 2000, pages 687 - 694, XP008086493

### SEQUENCE LISTING

<110> F. Hoffmann-La Roche AG
   <120> IPF-1 as a marker/target for beta-cell failure
   <130> case 22665
<160> 1
<170> PatentIn version 3.2
<210> 1
   <211> 283
   <212> PRT
   <213> Homo sapiens
<220>
   <221> Insulin Promoter Fcator 1
   <222> (1)..(283)
   <223> accession No. P52945
<400> 1

## Description

Type 2 diabetes is a disease of fast growing worldwide importance and can be described as a failure of the pancreatic beta-cell (beta-cell failure) to compensate, with enhanced insulin secretion of the beta-cells, for peripheral insulin resistance. This failure is explained by both a relative loss of beta-cell mass as well as secretory defects that include enhanced basal insulin secretion by the beta-cells and a selective loss of sensitivity to insulin mainly in skeletal muscle but also in other organs. The loss of beta-cell function is believed to be triggered by long-term exposure to enhanced levels of glucose and lipids (glyco- and lipotoxicity).

There is currently no clinically proven treatment that could prevent or delay beta-cell failure under lipo/glycotox conditions. It would also be useful to identify better targets for treatment and markers for detection of beta-cell failure or function that are more sensitive or more reliable than the markers commonly used, such as insulin, proinsulin or C-peptide.

Furthermore, it would be an advantage to identify markers that can be detected in plasma.

### Description of invention

The aim of the present invention is to identify and provide a novel method to screen for compounds that prevent, attenuate, or inhibit beta-cell failure, and for a marker that allows for diagnosis of beta-cell failure at an earlier stage of type II diabetes and more reliably than can presently be done.

Surprisingly, it was found that the use of protein IPF-1 can overcome, at least in part, the problems known from the state of the art.

Insulin Promoter Factor-1 (IPF-1) is a homeodomain-containing transcription factor critically required for the embryonic development of the pancreas and for the transcriptional regulation of endocrine pancreas-specific genes in adults, such as insulin. Mutations leading to a defect of IPF-1 function have been linked to type 2 diabetes (Macfarlane et al., 1999, J. Clin. Invest. 104, R33-R39).

Surprisingly, it was found that increased changes in the levels of secreted IPF-1 are found in beta-cell failure. The present invention provides an in vitro method of screening for a compound that prevents and/or inhibits and/or delays beta-cell failure, and a novel marker for the early diagnosis of beta-cell failure in diabetes.

In preferred embodiments, the novel marker IPF-1 may be used for diagnostic as well as for screening purposes.

In a preferred embodiment, the diagnostic method according to the present invention is used for patient screening purposes. I.e., it is used to assess subjects without a prior diagnosis of diabetes by measuring the level of IPF-1 in a bodily fluid sample and correlating the level of IPF-1 to the presence or absence of beta-cell failure.

The present invention also provides a method of screening for a compound that prevents and/or inhibits and/or delays beta-cell failure, comprising the step of detecting soluble IPF-1 secreted from a host in the presence or absence of said compound, wherein a compound that prevents and/or inhibits and/or delays beta-cell failure is a compound with which the level of IPF-1 secreted from a host is changed.

A host may be a model cell representing beta-cells in culture, or an animal which can be used as a model for beta-cell failure.

The present invention also provides for a use of protein IPF-1 as a marker for screening for a compound that prevents and/or inhibits beta-cell failure.

The diagnostic and patient screening methods according to the present invention are based on a bodily fluid sample which is derived from an individual. Unlike to methods known from the art IPF-1 is specifically measured from this bodily fluid sample by use of a specific binding agent.

A specific binding agent is, e.g., a receptor for IPF-1 or an antibody to IPF-1. As the skilled artisan will appreciate the term specific is used to indicate that other biomolecules present in the sample do not significantly bind to the binding agent specific for IPF-1. A level of less than 5% cross-reactivity is considered not significant.

A specific binding agent preferably is an antibody reactive with IPF-1. The term antibody refers to a polyclonal antibody, a monoclonal antibody, fragments of such antibodies, as well as to genetic constructs comprising the binding domain of an antibody.

Antibodies are generated by state of the art procedures, e.g., as described in Tijssen (Tijssen, P., Practice and theory of enzyme immunoassays 11 (1990) the whole book, especially pages 43-78; Elsevier, Amsterdam). For the achievements as disclosed in the present invention polyclonal antibodies raised in rabbits have been used. However, clearly also polyclonal antibodies from different species, e.g. rats or guinea pigs, as well as monoclonal antibodies can also be used. Since monoclonal antibodies can be produced in any amount required with constant properties, they represent ideal tools in development of an assay for clinical routine.

As the skilled artisan will appreciate now, that IPF-1 has been identified as a marker which is useful in the diagnosis of beta-cell failure, alternative ways may be used to reach a result comparable to the achievements of the present invention. For example, alternative strategies to generate antibodies may be used. Such strategies comprise amongst others the use of synthetic peptides, representing an epitope of IPF-1 for immunization. Alternatively, DNA immunization also known as DNA vaccination may be used.

For measurement the bodily fluid sample obtained from an individual is contacted with the specific binding agent for IPF-1 under conditions appropriate for formation of a binding agent IPF-1-complex. Such conditions need not be specified, since the skilled artisan without any inventive effort can easily identify such appropriate incubation conditions.

As a final step according to the methods disclosed in the present invention the amount of complex is measured and correlated to the diagnosis of beta-cell failure or to the respective control as hereinbefore described. As the skilled artisan will appreciate there are numerous methods to measure the amount of the specific binding agent IPF-1-complex all described in detail in relevant textbooks (cf., e.g., Tijssen P., supra, or Diamandis, et al., eds. (1996) Immunoassay, Academic Press, Boston).

Preferably IPF-1 is detected in a sandwich type assay format. In such assay a first specific binding agent is used to capture IPF-1 on the one side and a second specific binding agent, which is labeled to be directly or indirectly detectable, is used on the other side.

As mentioned above, it has surprisingly been found that IPF-1 can be measured from a bodily fluid sample obtained from an individual sample. No tissue and no biopsy sample are required to apply the marker IPF-1 in the diagnosis of beta-cell failure.

In a preferred embodiment the method according to the present invention is practiced with serum as liquid sample material.

In a further preferred embodiment the method according to the present invention is practiced with plasma as liquid sample material.

In a further preferred embodiment the method according to the present invention is practiced with whole blood as liquid sample material.

Whereas application of routine proteomics methods to tissue samples, leads to the identification of many potential marker candidates for the tissue selected, the inventors of the present invention have surprisingly been able to detect protein IPF-1 in a bodily fluid sample. Even more surprising they have been able to demonstrate that the presence of IPF-1 in such liquid sample obtained from an individual can be correlated to the diagnosis of beta-cell failure.

Antibodies to IPF-1 with great advantage can be used in established procedures, e.g., to beta-cell failure in situ, in biopsies, or in immunohistological procedures.

Preferably, an antibody to IPF-1 is used in a qualitative (IPF-1 present or absent) or quantitative (IPF-1 amount is determined) immunoassay.

Measuring the level of protein IPF-1 has proven very advantageous in the field of beta-cell failure and diabetes. Therefore, in a further preferred embodiment, the present invention relates to use of protein IPF-1 as a marker molecule in the diagnosis of beta-cell failure from a bodily fluid sample obtained from an individual.

The term marker molecule is used to indicate that changes in the level of the analyte IPF-1 as measured from a bodily fluid of an individual marks the presence of beta-cell failure.

It is preferred to use the novel marker IPF-1 in the early diagnosis of type II diabetes.

It is especially preferred to use the novel marker IPF-1 in the early diagnosis of glucose intolerance.

The use of protein IPF-1 itself, represents a significant progress to the challenging field of beta-cell failure diagnosis. Combining measurements of IPF-1 with other known markers for diabetes, like insulin, or with other markers of beta-cell failure yet to be discovered, leads to further improvements. Therefore in a further preferred embodiment the present invention relates to the use of IPF-1 as a marker molecule for diabetes, preferably for beta-cell failure, in combination with another marker molecule for diabetes, preferably for beta-cell failure, in the diagnosis of diabetes, preferably of beta-cell failure from a bodily fluid sample obtained from an individual. Preferred selected other diabetes markers with which the measurement of beta-cell failure may be combined are insulin, pre-insulin, and/or C-peptide.

Diagnostic reagents in the field of specific binding assays, like immunoassays, usually are best provided in the form of a kit, which comprises the specific binding agent and the auxiliary reagents required to perform the assay.

One way of assessing clinical utility of the novel marker IPF-1 is by measuring its levels in 10 diabetic patients depending on injections of exogenous insulin and comparing the levels with those measured in 10 patients with demonstrated normal beta-cell function. For statistical analysis, standard Student's t-test evaluation is performed with values < 0.05 being taken as significant.

Accuracy of a test can be described by its receiver-operating characteristics (ROC) (see especially Zweig, M. H., and Campbell, G., Clin. Chem. 39 (1993) 561-577). The ROC graph is a plot of all of the sensitivity/specificity pairs resulting from continuously varying the decision threshold over the entire range of data observed.

The clinical performance of a laboratory test depends on its diagnostic accuracy, or the ability to correctly classify subjects into clinically relevant subgroups. Diagnostic accuracy measures the test's ability to correctly distinguish two different conditions of the subjects investigated. Such conditions are for example health and disease.

In each case, the ROC plot depicts the overlap between the two distributions by plotting the sensitivity versus 1 - specificity for the complete range of decision thresholds. On the y-axis is sensitivity, or the true-positive fraction [defined as (number of true-positive test results) (number of true-positive + number of false-negative test results)]. This has also been referred to as positivity in the presence of a disease or condition. It is calculated solely from the affected subgroup. On the x-axis is the false-positive fraction, or 1 - specificity [defined as (number of false-positive results)/(number of true-negative + number of false-positive results)]. It is an index of specificity and is calculated entirely from the unaffected subgroup. Because the true- and false-positive fractions are calculated entirely separately, by using the test results from two different subgroups, the ROC plot is independent of the prevalence of disease in the sample. Each point on the ROC plot represents a sensitivity/-specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. (If the ROC plot falls completely below the 45° diagonal, this is easily remedied by reversing the criterion for "positivity" from "greater than" to "less than" or vice versa.) Qualitatively, the closer the plot is to the upper left corner, the higher the overall accuracy of the test.

One convenient goal to quantify the diagnostic accuracy of a laboratory test is to express its performance by a single number. The most common global measure is the area under the ROC plot. By convention, this area is always ≥ 0.5 (if it is not, one can reverse the decision rule to make it so). Values range between 1.0 (perfect separation of the test values of the two groups) and 0.5 (no apparent distributional difference between the two groups of test values). The area does not depend only on a particular portion of the plot such as the point closest to the diagonal or the sensitivity at 90% specificity, but on the entire plot. This is a quantitative, descriptive expression of how close the ROC plot is to the perfect one (area = 1.0).

The following examples, references and sequence listing are provided to aid the understanding of the present invention, the true scope of which is set forth in the appended claims. It is understood that modifications can be made in the procedures set forth.

### Examples

In order to identify proteins secreted by INS-1 (Asfari M, Janjic D, Meda P, Li G, Halban PA, Wollheim CB. Establishment of 2-mercaptoethanol-dependent differentiated insulin-secreting cell lines. Endocrinology. 1992 Jan;130(1):167-78) or RINm5f insulinoma cells (Praz GA, Halban PA, Wollheim CB, Blondel B, Strauss AJ, Renold AE. Regulation of immunoreactive-insulin release from a rat cell line (RINm5F). Biochem J. 1983 Feb 15;210(2):345-52) we applied two methods: (i) Fractionation of the cells by differential sedimentation into sub-cellular compartments with subsequent identification of the proteins based on their peptide mass fingerprint using MALDI-TOF mass spectrometry, and (ii) enrichment of glycoproteins by heparin chromatography followed by one-dimensional SDS-PAGE and identification of proteins by analysis of the tryptic peptides resulting from protein digest by liquid chromatography coupled to tandem mass spectrometry resulting in identification based on protein sequence tags. The combination of these two purification strategies allowed us to increase the efficiency of protein identification in the cellular compartments as well as in the medium of cultured cells.

### Cell culture

We reproduced the features of beta-cell failure by chronic exposure of beta-cells to a combination of high glucose/fatty acids (FAs), suggesting that hyperlipidemia as well as hyperglycemia may contribute to decompensation of beta-cells. INS-1E and RINm5f cells pretreated for 24 h with a combination of 10 mM glucose and 0.5 mM palmitate were used for these experiments.

### Example 1

### Mapping and identification of signal proteins in cell compartment by 2DE-Electrophoresis and identification by MALDI-MS

Samples prepared from each cell line were subjected to 2-DE as described elsewhere (Peyrl A, Krapfenbauer K, Slavc I, et al., PROTEOMICS 3 (9): 1781-1800 SEP 2003; Fountoulakis M., Langen H., Anal. Biochem. 250 (1997) 153-156.). 2-DE was performed essentially as reported (Langen, H., Roeder, D., Juranville, J.-F., Fountoulakis, M., Electrophoresis 1997, 18, 2085-2090). Samples were desalted by using membrane filter tubes (Millipore, Art. No. UFV4BGC25) and 2.0 mg were applied on immobilised pH 3-10 non linear gradient strips (Amersham, Pharmacia Biotechnology, Uppsala, Sweden) at both the basic and acid ends of the strips. The proteins were focused at 200 V after which the voltage is gradually increasing to 5000 V with 2 V/min. Focusing was continued at 5000 V for 24 h. The second-dimensional separation was performed on a 12% polyacrylamide gel (Biosolve, Walkinswaard, Netherland). The gels (180 x 200 x 1.5 mm) were run at 50 mA /gels, in an Ettan DALT II system (Amersham, Pharmacia Biotechnology, Uppsala, Sweden) accommodating twelve gels. After protein fixation for 12 h in 50% methanol containing 5% phosphoric acid, the gels were stained with colloidal Coomassie blue (Novex, San Diego, CA) for 24 h. Molecular masses were determined by running standard protein markers (Gibco, Basel, Switzerland), covering the range of 10 to 200 kDa. PI values were used as given by supplier of the IPG strips (Amersham Pharmacia, Uppsala, Sweden). Gels were destained with H₂O and scanned in an AGFA DUOSCAN densitometer. Electronic images of the gels were recorded using Photoshop (Adobe) and PowerPoint (Microsoft).

*MALDI-MS*: MS analysis was performed as described (Langen, H., Roeder, D., Juranville, J.-F., Fountoulakis, M., Electrophoresis 1997, 18, 2085-2090) with minor modifications.

Briefly, spots were excised, destained with 30% (v/v) acetonitrile in 0.1 M ammonium bicarbonate and dried in a Speed vac evaporator. The dried gel pieces were reswollen with 5 µl of 5 mM ammonium bicarbonate, (pH 8.8) containing 50 ng trypsin (Promega, Madison, WI, USA) were added, centrifuged for 1 min and left at room temperature for about 12 h. After digestion, 5 µl of water was added, followed 10 min later by 10 µl 75% acetonitrile, containing 0.3% trifluoroacetic acid, was added, centrifuged for 1 min and the content was vortexed for 20 min. For MALDI-MS 1.5 µl from the separated liquid was mixed with 1 µl saturated alpha-cyano cinnamic acid in 50% acetonitril, 0.1% TFA in water and applied to the MALDI target. The samples were analysed in a time-of-flight mass spectrometer (Ultraflex, Bruker, Bremen, Germany) equipped with a reflector and delayed extraction. Des-Arg-1 Bradykinin (Sigma) and ACTH (18-38) (Sigma) were used as standard peptides. Calibration was internal to the samples. The peptide masses were matched with the theoretical peptide masses of all proteins from all species of the SWISS-Prot database.

**Peak annotation for MALDI mass spectra:** Mass spectrometric data is two times filtered using a low-pass median parametric spline filter in order to determine the instrument baseline. The smoothed residual mean standard deviation from the baseline is used as an estimate of the instrument noise level in the data. After baseline correction and rescaling of the data in level-over-noise coordinates, the data point with the largest deviation from the baseline is used to seed a non-linear (Levenberg-Marquardt) data fitting procedure to detect possible peptide peaks. Specifically, the fit procedure attempts to produce the best fitting average theoretical peptide isotope distribution parameterized by peak height, resolution, and monoisotopic mass. The convergence to a significant fit is determined in the usual way by tracking sigma values. After a successful convergence, an estimate for the errors of the determined parameters is produced using a bootstrap procedure using sixteen repeats with a random exchange of 1/3 of the data points. The resulting fit is subtracted from the data, the noise level in the vicinity of the fit is adjusted to the sum of the extrapolated noise level and the deviation from the peak fit, and the process is iterated to find the next peak as long as a candidate peak more than five times over level of noise can be found. The process is stopped when more than 50 data peaks have been found. The zero and first order of the time-of-flight to mass conversion are corrected using linear extrapolation from detected internal standard peaks, and confidence intervals for the monoisotopic mass values are estimated form the mass accuracies of the peaks and standards.

**Probabilistic matching of spectra peaks to in-silico protein digests:** Peak mass lists for mass spectra are directly compared to theoretical digests for whole protein sequence databases. For each theoretical digest, [1-Π(1- N P(pi))]^{cMatches} is calculated, where *N* is the number of peptides in the theoretical digest, *P(pi)* is the number of peptides that match the confidence interval for the monoisotopic mass of the peak divided by the count of all peptides in the sequence database, and *cMatches* is the number of matches between digest and mass spectrum. It can be shown that this value is proportional to the probability of obtaining a false positive match between digest and spectrum. Probability values are further filtered for high significance of the spectra peaks that produce the matches. After a first round of identifications, deviations of the identifications for mass spectra acquired under identical conditions are used to correct the second and third order terms of the time-of-flight to mass conversion. The resulting mass values have mostly absolute deviations less than 10ppm. These mass values are then used for a final round of matching, where all matches having a *Pₘᵢₛₘ* less than 0.01/NProteins (1% significance level with Bonferoni correction) are accepted.

### Example 2

### Enrichment of putative secreted proteins by heparin columns from the medium and identification by LC-MS

Based on the observation that most of the proteins with a signal function are glycosylated, the nature of Heparin Sepharose columns makes it a very versatile tool for the separation of many glycosylated proteins like e.g. proteins with signal function, growth factors, coagulation proteins and steroid receptors. The ligand in the Heparin Sepharose column is a naturally occurring sulfated glycosaminglycan which is extracted from native proteoglycan of porcine intestinal mucosa. Heparin consists of alternating units of uronic acid and D-glucosamine, most of which are substituted with one or two sulfate groups. Immobilized heparin has two main modes of interaction with proteins. It can operate as an affinity ligand; e.g. in its interaction with coagulation factors. Heparin has also a function as a high capacity cation exchanger due to its anionic sulphate groups. In our case the column was operated by using a syringe.

Recommended elution conditions for both cases comprised increasing the ionic strength by using a step gradient of 2M NaCl in which the Binding buffer was 10 mM sodium phosphate pH ∼7, and the Elution buffer was 10 mM sodium phosphate, 2 M NaCl, pH ∼7.

### Sample preparation

25 ml of the medium were centrifuged at 10.000 g, for 10 min at 4°C in order to remove cells and other insoluble materials. The sample solution was adjusted to the composition of the binding buffer. This was done by diluting the sample by adding 25 ml of a 20 mM sodium phosphate buffer solution (pH = 7). The sample was centrifuged immediately before applying it on the column. The offloading volume for the Heparin Column (HiTrap Heparin HP, 1 ml, Cat.Nr. 17-0406-01, Amersham) was 5 ml for 1 ml column.

### Operation procedure for enrichment of proteins by Heparin chromatography:

1. A 25 ml syringe was filled with binding buffer. In addition to this the stopper was removed and the column was connected to the syringe with the provided adapter "drop to drop" to avoid introducing air into column.
2. The twist-off end was removed and in order to equilibrate the column, the heparin sepharose was washed with 10 column volumes of binding buffer.
3. The sample was then prepared as described above and applied by using a syringe fitted to the luer adaptor by pumping onto the column.
4. Then, the column was washed with 5 volumes of binding buffer or until no material appeared in the effluent.
5. To elute the sample, the column was washed with 5 column volumes of elution buffer by using a step gradient.
6. Finally, the purified fractions were desalted by using POROS R2 columns.

Sample fractions eluted from the Heparin column were desalted by using reversed phase chromatography (POROS R2, PerSeptive Biosytems), and dried by using a speed vac. After drying, samples were dissolved in sample buffer mentioned below and the protein content was determined by the Bradford procedure (BioRad potein assay, BioRad).

### 1D Electrophoresis

### Sample Loading and Running Conditions

15 µg of sample were dissolved in 20 µl Sample buffer (Sample, 2.5 µl NuPAGE LDS Sample Buffer (4X), 1.0 µl NuPAGE Reducing Agent (10X), and deionized water to 6.5µl, for a total volume of 10 µl) and, before applying onto the gel, heated at 70°C for 10 minutes. The upper buffer chamber was filled with 200 ml 1X NuPAGE SDS running buffer (MES SDS running Buffer was prepared by adding 50 ml of 20X NuPAGE MES SDS Running Buffer to 950 ml deionised water). As a reducing agent, 200 µl/200 ml of the antioxidant solution was added in the upper buffer chamber. Finally, the lower buffer chamber was filled with 600 ml 1X NuPAGE SDS running buffer and gel electrophoresis was performed on a 10% BT linear gradient, polyacrylamide gels (NuPAGE, Invitrogen) at constant 200 V at RT for 35 min.

### Staining and Destaining Procedure

After protein fixing with 50% (v/v) methanol containing 5% (v/v) phosphoric acid for 12 h, the gels were stained with colloidal Coomassie blue (Novex, San Diego, CA, USA) for further 24 h. The gels were destained with H₂O and scanned in a standard flatbed scanner. The images were processed using Photoshop (Adope) and PowerPoint (Microsoft) software. Protein bands were quantified using the Image Master 2D Elite software (Amersham Pharmacia Biotechnology).

LC-MS: For identification of secreted proteins our proteomics studies were also performed using an LC/MS system named multidimensional protein identification technology (MudPIT), which combines multidimensional liquid chromatography with electro-spray ionization tandem mass spectrometry. In order to separate the digested proteins enriched by Heparin columns, our multidimensional liquid chromatography method integrates a strong cation-exchange (SCX) resin and reversed-phase resin in a biphasic column. Each MudPIT analysis was done in duplicate and separation was reproducible within 0.5% between two analyses. Furthermore, a dynamic range of 10000 to 1 between the most abundant and least abundant proteins/peptides in a complex peptide mixture has been demonstrated. By improving sample preparation along with separations, the method improved the overall analysis of proteomes by identifying proteins of a fraction enriched with secreted proteins. The MudPIT system included a 4 cm × 50 - µm i.d. × 5 µm C18 microSPE pre-column for sample concentration and an 85 cm × 15-µm i.d. × 3 µm C18 packed capillary column for high efficiency gradient reversed-phase nanoscale LC separation of extremely small sample quantities. The microSPE stage allowed solution to be loaded onto the nanoLC column at approximately 8 µL min⁻¹ which required <2 min to load a 10 µL solution with a sample loss of <5% (due to the syringe and valve adapters). The separation is conducted at a constant pressure of 10,000 psi. The long 3-µm particle packed 15-µm-i.d. capillary provides a separation peak capacity of approximately 10³. The column is connected by a zero dead volume stainless steel union fitting to a replaceable nanoESI emitter made from a 10-µm -i.d. × 150- µm-o.d. fused silica capillary with an approximately 2-µm- i.d orifice for highly efficient ionization of the eluting peptide. The ESI source is interfaced to either an FTICR MS or an ion trap MS/MS for peptide/protein detection and identification. An FTICR mass spectrometer was used for single-stage MS based upon high-accuracy mass measurements and the use of relative retention time (RRT) information, and a Finnigan ion trap mass spectrometer (LCQ XP, ThermoQuest Corp., San Jose, CA) was used for MS/MS.

### Example 3

### Generation of antibodies to the beta-cell failure marker IPF-1

Polyclonal antibody to the beta-cell failure marker IPF-1 is generated for further use of the antibody in the measurement of serum and plasma and blood levels of IPF-1 by immunodetection assays, e.g. Western Blotting and ELISA.

### Recombinant protein expression in E. coli

In order to generate antibodies to IPF-1, recombinant expression of the protein is performed for obtaining immunogens. The expression is done applying a combination of the RTS 100 expression system and E.coli. In a first step, the DNA sequence is analyzed and recommendations for high yield cDNA silent mutational variants and respective PCR-primer sequences are obtained using the "ProteoExpert RTS E.coli HY" system. This is a commercial web based service (www.proteoexpert.com). Using the recommended primer pairs, the "RTS 100 E. coli Linear Template Generation Set, His-tag" (Roche Diagnostics GmbH, Mannheim, Germany, Cat.No. 3186237) system to generate linear PCR templates from the cDNA and for in-vitro transcription and expression of the nucleotide sequence coding for the IPF-1 protein is used. For Western-blot detection and later purification, the expressed protein contains a His-tag. The best expressing variant is identified. All steps from PCR to expression and detection are carried out according to the instructions of the manufacturer. The respective PCR product, containing all necessary T7 regulatory regions (promoter, ribosomal binding site and T7 terminator) is cloned into the pBAD TOPO^{®} vector (Invitrogen, Karlsruhe, Germany, Cat. No. K 4300/01) following the manufacturer's instructions. For expression using the T7 regulatory sequences, the construct is transformed into E. coli BL 21 (DE 3) (Studier, F.W., et al., Methods Enzymol. 185 (1990) 60-89) and the transformed bacteria are cultivated in a 1 l batch for protein expression.

Purification of His-IPF-1 fusion protein is done following standard procedures on a Ni-chelate column. Briefly, 1 l of bacteria culture containing the expression vector for the His-IPF-1 fusion protein is pelleted by centrifugation. The cell pellet is resuspended in lysis buffer, containing phosphate, pH 8.0, 7 M guanidium chloride, imidazole and thioglycerole, followed by homogenization using a Ultra-Turrax^{®}. Insoluble material is pelleted by high speed centrifugation and the supernatant is applied to a Ni-chelate chromatographic column. The column is washed with several bed volumes of lysis buffer followed by washes with buffer, containing phosphate, pH 8.0 and Urea. Finally, bound antigen is eluted using a phosphate buffer containing SDS under acidic conditions.

### Production of monoclonal antibodies against the protein IPF-1

### a) Immunization of mice

12 week old A/J mice are initially immunized intraperitoneally with 100 µg IPF-1. This is followed after 6 weeks by two further intraperitoneal immunizations at monthly intervals. In this process each mouse is administered 100 µg IPF-1 adsorbed to aluminum hydroxide and 10⁹ germs of *Bordetella pertussis*. Subsequently the last two immunizations are carried out intravenously on the 3rd and 2nd day before fusion using 100 µg IPF-1 in PBS buffer for each.

### b) Fusion and cloning

Spleen cells of the mice immunized according to a) are fused with myeloma cells according to Galfre, G., and Milstein, C., Methods in Enzymology 73 (1981) 3-46. In this process ca. 1*10⁸ spleen cells of the immunized mouse are mixed with 2x10⁷ myeloma cells (P3X63-Ag8-653, ATCC CRL1580) and centrifuged (10 min at 300 g and 4°C.). The cells are then washed once with RPMI 1640 medium without fetal calf serum (FCS) and centrifuged again at 400 g in a 50 ml conical tube. The supernatant is discarded, the cell sediment is gently loosened by tapping, 1 ml PEG (molecular weight 4000, Merck, Darmstadt) is added and mixed by pipetting. After 1 min in a water-bath at 37°C., 5 ml RPMI 1640 without FCS is added drop-wise at room temperature within a period of 4-5 min. Afterwards 5 ml RPMI 1640 containing 10% FCS is added drop-wise within ca. 1 min, mixed thoroughly, filled to 50 ml with medium (RPMI 1640+10% FCS) and subsequently centrifuged for 10 min at 400 g and 4°C. The sedimented cells are taken up in RPMI 1640 medium containing 10% FCS and sown in hypoxanthine-azaserine selection medium (100 mmol/l hypoxanthine, 1 µg/ml azaserine in RPMI 1640+10% FCS). Interleukin 6 at 100 U/ml is added to the medium as a growth factor. After ca. 10 days the primary cultures are tested for specific antibody. IPF-1-positive primary cultures are cloned in 96-well cell culture plates by means of a fluorescence activated cell sorter. In this process again interleukin 6 at 100 U/ml is added to the medium as a growth additive.

### c) Immunoglobulin isolation from the cell culture supernatants

The hybridoma cells obtained are sown at a density of 1x10⁵ cells per ml in RPMI 1640 medium containing 10% FCS and proliferated for 7 days in a fermenter (Thermodux Co., Wertheim/Main, Model MCS-104XL, Order No. 144-050). On average concentrations of 100 µg monoclonal antibody per ml are obtained in the culture supernatant. Purification of this antibody from the culture supernatant is carried out by conventional methods in protein chemistry (e.g. according to Bruck, C., et al., Methods in Enzymology 121 (1986) 587-695).

### Generation of polyclonal antibodies

### a) Immunization

For immunization, a fresh emulsion of the protein solution (100 µg/ml protein IPF-1) and complete Freund's adjuvant at the ratio of 1:1 is prepared. Each rabbit is immunized with 1 ml of the emulsion at days 1, 7, 14 and 30, 60 and 90. Blood is drawn and resulting anti-IPF-1 serum used for further experiments as described in examples 3 and 4.

### b) Purification of IgG (immunoglobulin G) from rabbit serum by sequential precipitation with caprylic acid and ammonium sulfate

One volume of rabbit serum is diluted with 4 volumes of acetate buffer (60 mM, pH 4.0). The pH is adjusted to 4.5 with 2 M Tris-base. Caprylic acid (25 µl/ml of diluted sample) is added drop-wise under vigorous stirring. After 30 min the sample is centrifuged (13,000 x g, 30 min, 4°C), the pellet discarded and the supernatant collected. The pH of the supernatant is adjusted to 7.5 by the addition of 2 M Tris-base and filtered (0.2 µm).

The immunoglobulin in the supernatant is precipitated under vigorous stirring by the drop-wise addition of a 4 M ammonium sulfate solution to a final concentration of 2 M. The precipitated immunoglobulins are collected by centrifugation (8000 x g, 15 min, 4°C).

The supernatant is discarded. The pellet is dissolved in 10 mM NaH₂PO₄/NaOH, pH 7.5, 30 mM NaCl and exhaustively dialyzed. The dialysate is centrifuged (13,000 x g, 15 min, 4°C) and filtered (0.2 µm).

### Biotinylation of polyclonal rabbit IgG

Polyclonal rabbit IgG is brought to 10 mg/ml in 10 mM NaH₂PO₄/NaOH, pH 7.5, 30 mM NaCl. Per ml IgG solution 50 µl Biotin -N-hydroxysuccinimide (3.6 mg/ml in DMSO) are added. After 30 min at room temperature, the sample is chromatographed on Superdex 200 (10 mM NaH₂PO₄/NaOH, pH 7.5, 30 mM NaCl). The fraction containing biotinylated IgG are collected. Monoclonal antibodies have been biotinylated according to the same procedure.

### Digoxygenylation of polyclonal rabbit IgG

Polyclonal rabbit IgG is brought to 10 mg/ml in 10 mM NaH₂PO₄/NaOH, 30 mM NaCl, pH 7.5. Per ml IgG solution 50 µl digoxigenin-3-O-methylcarbonyl-ε-aminocaproic acid-N-hydroxysuccinimide ester (Roche Diagnostics, Mannheim, Germany, Cat. No. 1 333 054) (3.8 mg/ml in DMSO) are added. After 30 min at room temperature, the sample is chromatographed on Superdex^{®} 200 (10 mM NaH₂PO₄/NaOH, pH 7.5, 30 mM NaCl). The fractions containing digoxigenylated IgG are collected. Monoclonal antibodies are labeled with digoxigenin according to the same procedure.

### Example 4

### Western Blot

Protein samples enriched and isolated from the medium by Heparin columns (mentioned above) were solved in sample buffer consisting of 10 mM Tris-HCl (pH 7.5), 150 mM NaCl, 0.05 % Tween 20, 1 % SDS, and centrifuged at 12,000 g for 10 min at 4°C. The protein concentration of the supernatant was measured by Bradford using a standard curve constructed from a range of known bovine serum albumin standards. After mixing samples with sample buffer (60 mM Tris-HCl, 2% SDS, 0.1% bromphenol blue, 25% glycerol, and 14.4 mM 2-mercaptoethanol, pH 6.8) and incubation at 70°C for 5 min, samples were separated by 12.5% homogenous ExcelGel SDS gels (Amersham Bioscience) and electro transferred onto Nitrocellulose membranes. After incubation in blocking solution (10 mM Tris-HCl, pH 7.5, 150 mM NaCl, 0.05% Tween 20 and 5% non-fat dry milk), membranes were incubated with rabbit anti-rat antibody for 2 hrs at room temperature, respectively. After washing 3 times for 10 min with washing solution (0.3% Tween 20 in tris-buffered saline), membranes were incubated with a horseradish peroxidase conjugated anti-rabbit IgG (H+L), anti-mouse IgG₁ and anti-mouse IgG₂ₐ (Southern Biotechnology Associates, Inc., Birmingham, AL), respectively, for 1 hr at room temperature. Membranes were washed 3 times for 10 min and antigen-antibody complexes were visualized by an enhanced chemiluminescence's reagent (Western Lightning ™, PerkinElmer Life Sciences, Inc., Boston, MA) on an X-ray film according to the manufacturer's protocol.

### Example 5

### ELISA for the measurement of IPF-1 in human serum and plasma samples.

For detection of IPF-1 in human serum or plasma, a sandwich ELISA is developed. For capture and detection of the antigen, aliquots of the anti-IPF-1 polyclonal antibody (see Example 2) are conjugated with biotin and digoxygenin, respectively.

Streptavidin-coated 96-well microtiter plates are incubated with 100 µl biotinylated anti-IPF-1 polyclonal antibody for 60 min at 10 µg/ml in 10 mM phosphate, pH 7.4, 1% BSA, 0.9% NaCl and 0.1% Tween 20. After incubation, plates are washed three times with 0.9% NaCl , 0.1% Tween 20. Wells are then incubated for 2 h with either a serial dilution of the recombinant protein (see Example 2) as standard antigen or with diluted plasma samples from patients. After binding of IPF-1, plates are washed three times with 0.9% NaCl , 0.1% Tween 20. For specific detection of bound IPF-1, wells are incubated with 100 µl of digoxygenylated anti-IPF-1 polyclonal antibody for 60 min at 10 µg/ml in 10 mM phosphate, pH 7.4, 1% BSA, 0.9% NaCl and 0.1% Tween 20. Thereafter, plates are washed three times to remove unbound antibody. In a next step, wells are incubated with 20 mU/ml anti-digoxigenin-POD conjugates (Roche Diagnostics GmbH, Mannheim, Germany, Catalog No. 1633716) for 60 min in 10 mM phosphate, pH 7.4, 1% BSA, 0.9% NaCl and 0.1% Tween 20. Plates are subsequently washed three times with the same buffer. For detection of antigen-antibody complexes, wells are incubated with 100 µl ABTS solution (Roche Diagnostics GmbH, Mannheim, Germany, Catalog No. 11685767) and OD is measured after 30-60 min at 405 nm with an ELISA reader.

### Example 6

### Statistical analysis of patient data:

Clinical utility of the novel marker IPF-1 is assessed by measuring its levels in 10 diabetic patients depending on injections of exogeneous insulin and comparing the levels with those measured in 10 patients with demonstrated normal beta cell function. Statistical analysis is performed by standard Student's t-test evaluation with values <0.05 taken as significant.

## Claims

1. An *in vitro* method of screening for a compound that prevents and/or inhibits and/or delays beta-cell failure, comprising the step of detecting soluble insulin promoter factor 1 (IPF-1) secreted from a host in the presence or absence of said compound, wherein a compound that prevents and/or inhibits and/or delays beta-cell failure is a compound with which the level of IPF-1 is secreted from a host is changed.

2. A method for the diagnosis of beta cell failure comprising the steps of
a) providing a bodily fluid sample obtained from an individual,
b) contacting said bodily fluid sample with a specific binding agent for IPF-1 under conditions appropriate for formation of a complex between said binding agent and IPF-1, and
c) correlating the amount of complex formed in (b) to the diagnosis of beta cell failure.

3. The methods according to any one of claim 2, further **characterized in that** said sample is serum.

4. The method according to claim 2, further **characterized in that** said sample is plasma.

5. The method according to claim 2, further **characterized in that** said sample is whole blood.

6. Use of a protein IPF-1 as a marker molecule in the diagnosis of beta-cell failure and/or in the early diagnosis of type II diabetes from a bodily fluid sample obtained from an individual.

7. The use of claim 6, wherein the protein IPF-1 is used in combination with at least one other marker molecule for beta-cell failure in the diagnosis of beta-cell failure from bodily fluid sample obtained from an individual.

8. The use according to claim 6, wherein the early diagnosis of type II diabetes is made with a sample derived from patients suffering from glucose intolerance.

## Patentansprüche

1. Ein *in vitro* Verfahren zur Selektion einer Verbindung, die Beta-Zellversagen verhindert und/oder hemmt und/oder verzögert, umfassend den Schritt des Nachweises von löslichem Insulin Promoter Faktor 1 (IPF-1), der von einem Wirt in Anwesenheit oder Abwesenheit der Verbindung sekretiert wird, wobei eine Verbindung, welche das Beta-Zellversagen verhindert und/oder hemmt und/oder verzögert, eine Verbindung ist, welche das von einem Wirt sekretierte IPF-1 Niveau ändert.

2. Ein Verfahren zur Diagnose des Beta-Zellversagens umfassend die Schritte:
(a) Bereitstellen einer Körperflüssigkeitsprobe, welche von einem Individuum gewonnen wurde.
(b) In Kontakt bringen der Körperflussigkeitsprobe mit einem spezifischen Bindungsmittel für IPF-1 unter geeigneten Bedingungen zur Bildung eines Komplexes zwischen dem Bindungsmittel und IPF-1, und
(c) Korrelation der Menge des in schritt (b) gebildeten Komplexes mit der Diagnose des Beta-Zellversagens.

3. Das Verfahren gemäss Anspruch 2, weiter **gekennzeichnet dadurch, dass** die Probe Serum ist.

4. Das Verfahren gemäss Anspruch 2, weiter **gekennzeichnet dadurch, dass** die Probe Plasma ist.

5. Das Verfahren gemäss Anspruch 2, weiter **gekennzeichnet dadurch, dass** die Probe Gesamtblut ist.

6. Verwendung eines IPF-1 Proteins als Markermolekül zur Diagnose des Beta-Zellversagens und/oder der Frühdiagnose von Diabetes Typ-2 aus einer Körperflüssigkeitsprobe, welche von einem Individuum gewonnen wurde.

7. Die Verwendung nach Anspruch 6, wobei das IPF-1 Protein in Verbindung mit mindestens einem anderen Markermolekül für das Beta-Zellversagen zur Diagnose des Beta-Zellversagens aus einer Körperflüssigkeitsprobe, welche aus einem Individuum gewonnen wurde, verwendet wird.

8. Die Verwendung nach Anspruch 6, wobei die Frühdiagnose von Diabetes Typ-2 mit einer Probe von Patienten, welche an Glukose-Intoleranz leiden, gemacht wird.

## Revendications

1. Procédé *in vitro* de criblage d'un composé qui empéche et/ou inhibe et/ou retarde la défaillance de cellules bêta, comprenant l'étape consistant à détecter le facteur promoteur d'insuline soluble 1 (IPF-1) sécrété par un hôte en présence ou en l'absence dudit composé, dans lequel un composé qui empêche et/ou inhibe et/ou retarde la défaillance de cellules bêta est un composé avec lequel le niveau de IPF-1 est sécrété par un hôte est modifié.

2. Procédé pour le diagnostic de la défaillance de cellules bêta comprenant les étapes consistant à
a) fournir un échantillon de fluide corporel obtenu chez un individu,
b) contacter ledit échantillon de fluide corporel avec un agent de liaison spécifique pour IPF-1 dans das conditions appropriées pour la formation d'un complexe entre ledit agent de liaison et IPF-1, et
c) corréler la quantité de complexe formé en (b) avec le diagnostic de défaillance de cellules bêta.

3. Procédé selon la revendication 2, **caractérisés de plus en ce que** l'échantillon est un sérum.

4. Procédé selon la revendication 2, **caractérisé de plus en ce que** l'échantillon est du plasma.

5. Procédé selon la revendication 2, **caractérisé de plus en ce que** l'échantillon est du sang complet.

6. Utilisation d'une protéine IPF-1 en tant que molécule marqueur dans le diagnostic de la défaillance de cellules bêta et/ou dans le diagnostic précoce de diabètes de type II à partir d'un échantillon de fluide corporel obtenu chez un individu.

7. Utilisation de la revendication 6, dans lequel la protéine IPF-1 est utilisée en combinaison avec au moins une autre molécule marqueur pour la défaillance de cellules bêta dans le diagnostic de la défaillance de cellules bêta à partir d'un échantillon de fluide corporel obtenu chez un

8. Utilisation selon la revendication 6, dans lequel le diagnostic précoce de diabètes de type II est analysé avec un échantillon dérivé de patients souffrant d'intolérance au glucose.
